# EUROPÄISCHE PATENTSCHRIFT

(11) **EP 3 997 504 B1**
(45) Veröffentlichungstag und Bekanntmachung des Hinweises auf die Patenterteilung: **09.10.2024**
(21) Anmeldenummer: 19721516.3
(22) Anmeldetag: 25.03.2019
(51) Int. Cl.: G02B 21/00, G02B 21/24, G02B 21/36, A61B 90/00, A61B 34/20, A61B 90/50, A61B 90/25, G02B 21/22, G02B 27/01, G02B 7/00

(54) **STEREOMIKROSKOP ZUR VERWENDUNG BEI MIKROCHIRURGISCHEN EINGRIFFEN AM PATIENTEN UND VERFAHREN ZUR STEUERUNG DES STEREOMIKROSKOPS**
STEREO MICROSCOPE FOR USE IN MICROSURGICAL OPERATIONS ON A PATIENT AND METHOD FOR CONTROLLING THE STEREO MICROSCOPE
STÉRÉOMICROSCOPE DESTINÉ À ÊTRE UTILISÉ LORS D'INTERVENTIONS MICROCHIRURGICALES CHEZ LE PATIENT ET PROCÉDÉ DE COMMANDE DU STÉRÉOMICROSCOPE

(30) Priorität: 26.03.2018 AT 812018
(43) Veröffentlichungstag der Anmeldung: 18.05.2022
(73) Patentinhaber: BHS Technologies GmbH, 6020 Innsbruck (AT)
(72) Erfinder: SANTEK, Michael, 6091 Götzens (AT); BURGER, Gregor, 6176 Völs (AT); CAPELLI, Mark, 6020 Innsbruck (AT); HÜTTER, Markus Friedrich, 6170 Zirl (AT)
(74) Vertreter: Prüfer & Partner mbB Patentanwälte · Rechtsanwälte
(86) Internationale Anmeldenummer: PCT/AT2019/000005
(87) Internationale Veröffentlichungsnummer: WO 2019/183648

(56) Entgegenhaltungen:
- EP-A2- 1 333 306
- DE-A1- 102015 218 926
- US-A1- 2005 063 047

## Beschreibung

### TECHNISCHES FELD

Die vorliegende Erfindung betrifft ein Stereomikroskop zur Verwendung bei mikrochirurgischen Eingriffen am Patienten sowie ein Verfahren zum Steuern des Stereomikroskops.

### HINTERGRUND DER ERFINDUNG

Auf dem Gebiet der Chirurgie an kleinsten Strukturen, beispielsweise in der Neurochirurgie oder Eingriffe an den Gehörknöchelchen im Mittelohr, sowie in der Implantationschirurgie ist die Mikroskop-unterstützte chirurgische Operation bekannt. Dabei wird die zu behandelnde Stelle, im folgenden Eingriffsbereich bezeichnet, am Patienten an der der mikrochirurgische Eingriff zu erfolgen hat mithilfe eines Mikroskops vergrößert dem Chirurgen dargestellt.

Aus US8005571 B2 ist in Robotersystem zur Verwendung bei chirurgischen Eingriffen bekannt. Das Robotersystem umfasst ein Stereomikroskop. Bei diesem System sitzt der Chirurg abseits vom Patienten vor einem Computer der das Robotersystem und Stereomikroskop steuert. Das Robotersystem umfasst die für den Eingriff zu verwendenden chirurgischen Werkzeuge und Arme mit denen diese Werkzeuge bewegt werden können. Damit ist das Stereomikroskop mit den die chirurgischen Werkzeug führenden Arme fest verbunden. Dem Chirurgen kann auf diese Weise immer ein Bild des Eingriffsbereichs präsentiert werden in dem die Arme sich von einer vorbestimmten Richtung, zumeist von unten, in den angezeigten Bildbereich hinein bewegen. Nachteilig bei einem derartigen System ist, dass der Chirurg sich weit vom Patienten entfernt befindet und so nur eingeschränkt einen gesamtheitlichen Eindruck des Eingriffs erfährt. Auch nachteilig ist bei diesem System, dass die Arme des Robotersystems kein so präzises Führen der chirurgischen Werkzeuge erlauben wie es die Hände des Chirurgen in der Lage sind zu tun.

Aus US20120190965 ist ein weiteres stereomikroskopisches System zur Verwendung bei Mikrochirurgischen Eingriffen bekannt. Das System ist vom Aufbau dem aus US8005571 B2 bekannten System vergleichbar aufgebaut und weist dieselben Nachteile auf. Auch hier ist durch die fixe Verbindung des Stereomikroskops mit den das chirurgische Werkzeug führenden Armen es nicht erforderlich eine Augen-Hand Koordination herzustellen. Ebenso nachteilig ist auch hier, dass sich die Arme mit den chirurgischen Werkzeugen nur von einer Seite her dem Eingriffsbereich nähern können.

Die Druckschrift US 2005/063047 A1 offenbart ein Mikroskopiesystem, welches mehrere Funktionen für einen Benutzer bereitstellt, die durch eine Positionierung oder Orientierung von Körperteilen des Benutzers einstellbar sind. Diese Funktionen umfassen unter anderem eine Positionierung eines eine Mikroskopieoptik halternden Stativs und eine Einstellung einer Stereobasis zur Erzeugung von stereoskopischen Darstellungen eines zu beobachtenden Objekts.

Es ist daher eine Aufgabe der vorliegenden Erfindungsmeldung ein Stereomikroskop 101 bereitzustellen, dass es dem Benutzer 103, d.h. dem Chirurgen, ermöglicht nicht nur ein stereoskopisches Bild des Eingriffsbereichs 117 bereitzustellen, sondern dass sich der Benutzer 103 zudem frei bewegen kann und unter jedem beliebigen Betrachtungswinkel es fortwährend gewährleistet ist, dass auch die Augen-Hand Koordination stereoskopisch dem natürlichen empfinden entspricht. Der Benutzer 103 kann dadurch den Eingriff am vergrößerten Eingriffsbereich 117 sicher und genau durchführen, sowie Vorteilhaft von jeder Seite her das chirurgische Werkzeug mit der Hand führen.

### ZUSAMMENFASSUNG DER ERFINDUNG

Diese Aufgabe wird mit dem erfindungsgemäßen Stereomikroskop durch die Merkmale des Anspruchs 1 erreicht. Vorteilhafte Weiterbindungen sind in den abhängigen Ansprüchen angegeben. Anspruch 15 gibt zudem das erfindungsgemäße Verfahren zur Steuerung des erfindungsgemäßen Stereomikroskops an.

Ein erfindungsgemäßes Stereomikroskop zur Verwendung bei mikrochirurgischen Eingriffen umfasst ein Stativ schwenkbar über ein Gelenk mit einem Roboterarm verbindbar, zwei mit dem Stativ derart verbindbare optische Bilderfassungseinheiten um ein stereoskopisches Bild des während der Benutzung abzubildenden Eingriffsbereichs am Patienten erfassen zu können und durch die zwei optischen Achsen der Bilderfassungseinheiten eine Bildaufnahmeebene definierend, eine Videobrille umfassend zwei optische Bildwiedergabeeinheiten mit je einer optischen Achse und einer Anzeige zur Wiedergabe eines Bildes welche gemeinsam eine Bildebene definieren, wobei die optischen Bildwiedergabeeinheiten derart angeordnet sind zur Erzeugung eines stereoskopischen Bildeindruckes einem die Videobrille tragenden Benutzers und durch die zwei optischen Achsen der optischen Bildwiedergabeeinheiten eine Bildwiedergabeebene definierend, eine Erfassungseinrichtung zur Ermittlung der räumlichen Orientierung der Videobrille, Bildwiedergabeebene, Bildebene und der Bildaufnahmeebene, eine Kontrolleinheit eingerichtet ein Kontrollsignal zum Verschwenken des Stativs bereitzustellen sodass die Schnittlinien in der Bildwiedergabeebene von der Bildebene und der Bildaufnahmeebene parallel gebracht werden können.

Das Kontrollsignal kann optisch in zumindest einer Bildwiedergabeeinheit eingeblendet werden und ist geeignet dem Benutzer die manuelle Verschwenkung des Stativs um das Gelenk anzuzeigen. In einer weiteren Ausführungsform kann das Stativ mit einer Motoreinheit um das Gelenk schwenkbar sein und das Kontrollsignal ausgebildet ist die Motoreinheit anzusteuern. In einer weiteren Ausführungsform können die zwei Bildwiedergabeeinheiten ein gemeinsames Display umfassen, dessen Anzeigebereich derart in zwei Hälften geteilt ist um je einem Auge des die Videobrille tragenden Benutzers ein Bild anzeigen zu können.

In einer vorteilhaften Weiterbildung umfasst die Erfassungseinheit zur Ermittlung der räumlichen Orientierung der Videobrille und/oder Bildaufnahmeebene ein optisches Objekterfassungssystem oder einen mit der Videobrille verbindbaren Orientierungssensor der bevorzugt aus der Gruppe Lagesensor, Positionssensor, Beschleunigungssensor oder ein Inertialmesssystem gewählt wird. Das erfindungsgemäße Stereomikroskop kann zusätzlich eine Kommunikationseinheit eingerichtet um in Echtzeit die stereoskopisch erfassten Bilder von den Bilderfassungseinheiten an die Bildwiedergabeeinheiten zur stereoskopischen Anzeige zu übermitteln aufweisen. Vorteilhafter weise beträgt der Zeitversatz in der Echtzeitkommunikation weniger als 50 Millisekunden. Die Übermittlung kann dabei per drahtloser Kommunikation erfolgen.

In einer vorteilhaften Weiterbildung kann das Stativ eine Schnellwechselplatte umfassen mit dem die Bildwiedergabeeinheiten verbindbar sind. In einer weiteren vorteilhaften Ausführungsform können die Bilderfassungseinheiten derart zueinander beweglich sein, um die stereoskopische Abbildung des Eingriffsbereichs abhängig von der Vergrößerung und/oder dem Abstand der Bilderfassungseinheiten vom Eingriffsbereich und/oder dem Augenabstand des Benutzers einzustellen. Die Bilderfassungseinheiten können dazu in der Bildaufnahmeebenen drehbar sein, sodass die optischen Achsen zueinander einen Winkel kleiner 180° einschließen. Oder den Abstand der beiden Bilderfassungseinheiten zueinander abhängig vom Abstand der Bilderfassungseinheiten vom Eingriffsbereich einstellen.

Das erfindungsgemäße Verfahren zum Steuern eines erfindungsgemäßen Stereomikroskops umfasst eine Kontrolleinheit die eingerichtet ist die folgenden Schritte auszuführen: Erfassung der Orientierung der Videobrille, Bildwiedergabeebene und Bildebene; Erfassung der Orientierung der Bildaufnahmeebene; Ermitteln einer ersten Schnittlinien von der Bildebene in der Bildwiedergabeebene; Ermitteln einer zweiten Schnittlinien von der Bildaufnahmeebene in der Bildwiedergabeebene; ermittelten und Ausgabe des ermittelten Kontrollsignals zum Verschwenken des Stativs, sodass die erste und zweite Schnittlinie parallel gebracht werden können.

### KURZE BESCHREIBUNG DER ZEICHUNGEN

Abbildung 1 zeigt ein mikrochirurgisches Stereomikroskop in einer bevorzugten Ausführungsform gemäß der Erfindung.
Abbildung 2 zeigt eine bevorzugte Ausführungsform des Stativs für das erfindungsgemäße Stereomikroskop.
Abbildung 3 zeigt eine Videobrille in einer für das erfindungsgemäße Stereomikroskop bevorzugten Ausführungsform.
Abbildung 4 zeigt ein Flussdiagramm zur Steuerung des erfindungsgemäßen Stereomikroskops.
Abbildung 5 zeigt schematisch das Display einer Bildwiedergabeeinheit mit der Überblendung durch ein beispielhaftes Kontrollsignal um dem Benutzer die manuelle Verschwenkung des Stativs anzuzeigen.

### BESCHREIBUNG DER BEVORZUGTEN AUSFÜHRUNGSFORMEN ANHAND DER ABBILDUNGEN

Im Folgenden werden bevorzugte Ausführungsformen des erfindungsgemäßen Stereomikroskops 100 zur Verwendung bei mikrochirurgischen Eingriffen sowie ein Verfahren zum Steuern des erfindungsgemäßen Stereomikroskops anhand der beigefügten Abbildungen beschrieben.

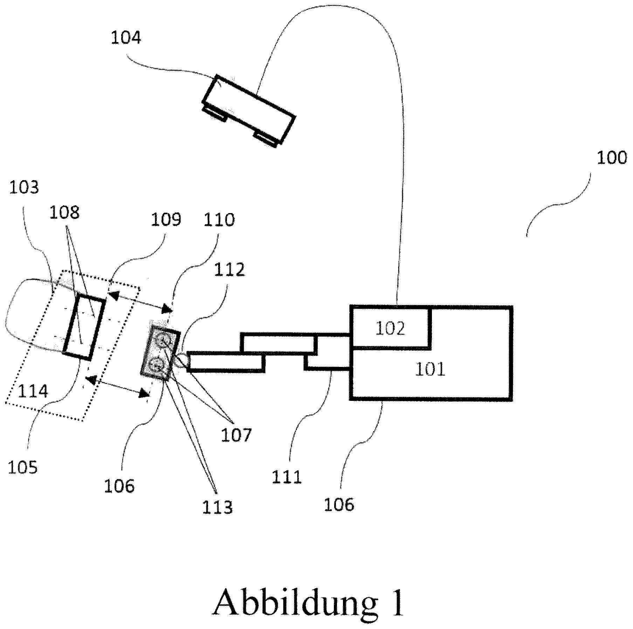

Abbildung 1 zeigt schematisch den Aufbau eines erfindungsgemäßen Stereomikroskops 100 in der Draufsicht zur Verwendung bei mikrochirurgischen Eingriffen. Das Stereomikroskop 100 umfasst ein Stativ 106 das schwenkbar über ein Gelenk 112 mit einem Roboterarm 111 verbindbar ist. Der Roboterarm 111 ist über eine Haltevorrichtung 106 stabil auf dem Boden des Operationssaales fixierbar um Schwingungen weitestgehend zu vermeiden. Mit dem Stativ 106 sind zwei optische Bilderfassungseinheiten 1 13 derart verbindbar um ein stereoskopisches Bild des während der Benutzung abzubildenden Eingriffsbereichs 1 17 am Patienten erfassen zu können und durch die zwei optischen Achsen 107 der Bilderfassungseinheiten 113 eine Bildaufnahmeebene 110 definierend. Die Bilderfassungseinheiten 113 sind eingerichtet um eine bis zu 100-fache optische Vergrößerung zu ermöglichen. In einer vorteilhaften Ausführungsform können die Bilderfassungseinheiten 1 13 zusätzlich dazu eingerichtet sein eine optische Verkleinerung zu ermöglichen. Das ist insbesondere für den Benutzer 103 von Vorteil, da er sich einen Überblick über den Eingriffsbereich 117 und darüber hinaus verschaffen kann. Auch hilft es dem Benutzer 103 sich zu orientieren, wenn er sich mit den chirurgischen Werkzeugen in den Händen dem Eingriffsbereich 117 nähert.

Die zwei optischen Bilderfassungseinheiten 113 können in einer beispielhaften Ausführungsform aus zwei Objektiven mit je einem digitalen Bilderfassungssystem, wie beispielsweise einer CCD-Kamera, ausgebildet sein. Derartige Bilderfassungseinheiten 113 sind aus dem Stand-der-Technik bekannt und weisen in bekannter Weise jeweils eine optische Achse 107 entlang des Objektivs und durch das rotationssymmetrische Zentrum der Objektivlinse verlaufend auf. Die beiden optischen Achsen 107 verlaufen in Abbildung 1 in die Bildebene hinein und der in der Draufsicht nicht dargestellte Eingriffsbereich 117 befindet sich unter den beiden Bilderfassungseinheiten 113. Die beiden optischen Achsen 107 liegen in einer Ebene, der Bildaufnahmeebene 110, welche rechtwinkelig zur Bildebene in Abbildung 1 verläuft und als Linie dargestellt ist, der Schnittlinie der Bildaufnahmeebene 110 mit der Bildebene der Abbildung 1. Die von den beiden Bilderfassungseinheiten 113 erfassten Bilder werden mit einer Videobrille 105 umfassend zwei optische Bildwiedergabeeinheiten 115 mit je einer optischen Achse 108 und einer Anzeige zur Wiedergabe eines Bildes, welche gemeinsam eine Bildebene 109 definieren, einem die Videobrille 105 tragenden Benutzer 103 übermittelt und angezeigt. Je eine Bildwiedergabeeinheit 115 stellt für je ein Auge des Benutzers 103 ein Bild bereit. Das erfasste Bild von je einer der beiden Bilderfassungseinheiten 107 wird damit von je eine der beiden Bildwiedergabeeinheiten 115 angezeigt. Damit wird erreicht, dass das stereoskopisch erfasste Bild des Eingriffsbereiches 1 17 je einem Auge des die Videobrille 105 tragenden Benutzers 103 angezeigt werden kann und dem Benutzer 103 ein stereoskopischer Bildeindruck des Eingriffsbereichs 117 vermittelt werden kann. Ein wesentlicher Vorteil des erfindungsgemäßen Stereomikroskops 100 gegenüber aus dem Stand-der-Technik bekannten besteht darin, dass der Benutzer 103 sich frei bewegen kann und den Eingriff von jeder beliebigen Richtung und mit beliebiger Kopfhaltung mit durch die Hände des Benutzers 103 geführten mikrochirurgischen Werkzeugen am Eingriffsbereich 117 vornehmen kann. Das binokulare Sehen des Menschen ist jedoch weitaus komplexer und es ist nicht ausreichend einen nur stereoskopischen Bildeindruck bereitzustellen, vor allem wenn durch den stereoskopischen Bildeindruck eine natürliche Augen-Hand Koordination dem Benutzer 103 zusätzlich ermöglicht werden sollte. Für mikrochirurgische Eingriffe ist eine korrekte Umsetzung der Augen-Hand Koordination unumgänglich, denn eine nur ganz geringfügige Fehl Führung der mit den Händen des Benutzers 103 geführten chirurgischen Werkzeuge kann zu schwerwiegenden Verletzungen im Eingriffsbereich 117 und in der Folge Komplikationen am Patienten führen.

Dazu umfasst das erfindungsgemäße Stereomikroskop 100 zusätzlich eine Erfassungseinrichtung 104 zur Ermittlung der räumlichen Orientierung der Videobrille 105, Bildwiedergabeebene 114, Bildebene 109 und der Bildaufnahmeebene 1 10. Die Kontrolleinheit 101 ist eingerichtet um ein Kontrollsignal zum Verschwenken des Stativs 106 bereitzustellen, sodass die Schnittlinien in der Bildwiedergabeebene 114 von der Bildebene 109 und der Bildaufnahmeebene 110 parallel gebracht werden können. Dadurch ist es möglich dem Benutzer 103 nicht nur ein stereoskopisches Bild des Eingriffsbereichs 117 bereitzustellen, der Benutzer 103 kann sich auch frei Bewegen und es ist fortwährend gewährleistet, dass auch die Augen-Hand Koordination stereoskopisch dem natürlichen empfinden entspricht. Der Benutzer 103 kann dadurch den Eingriff am vergrößerten Eingriffsbereich 117 sicher und genau durchführen. Das erfindungsgemäße Stereomikroskop 100 kann unterschiedliche Bewegungsmodi aufweisen. Beispielsweise kann in einem Bewegungsmodus die Bildaufnahmeebene 110 um den Fokuspunkt (Bezugspunkt) im Eingriffsbereichs 117 rotiert werden, wenn der die Videobrille 105 tragende Benutzer 103 den Kopf rotiert. Das erfindungsgemäße Stereomikroskop 100 funktioniert mit allen möglichen Bewegungsmodi und damit einhergehend frei definierbare Bezugspunkte und ist keinesfalls in irgendeiner Weise eingeschränkt.

Als Erfassungseinrichtung 104 ist jede aus dem Stand-der-Technik bekannte Vorrichtung geeignet, die die räumliche Orientierung und in einer weiteren Ausführungsform zusätzlich die Position von Objekten ermitteln kann. In einer bevorzugten Ausführungsform umfasst die Erfassungseinrichtung 104 ein optisches System, dass beispielsweise zunächst die räumliche Orientierung der Videobrille 105 durch an vorbekannten Punkten der Videobrille 105 angebrachten Markierungen ermittelt. Die Markierungen können beispielsweise farbig oder im infraroten Bereich reflektierende Punkte, Linien oder andere per Bilderkennung erkennbare Symbole sein. Die räumliche Orientierung der Bildwiedergabeebene 114 und der Bildebene 109, deren Lage relativ zur Videobrille 105 vorbekannt ist, kann dann mittels herkömmlicher linearer Transformationsmatrizen aus der räumlichen Orientierung der Videobrille 105 ermittelt werden. Diese Funktionalität der Erfassungseinrichtung 104 kann Teil der Kontrolleinheit 101 sein, die in diesem Fall dazu eingerichtet ist um aus der räumlichen Orientierung der Videobrille 105 die räumliche Orientierung der Bildwiedergabeebene 114 zu ermitteln. Damit kann in vorteilhafterweise die Rechenleistung der Kontrolleinheit 101 für die Berechnung der Transformation genutzt werden und die Erfassungseinrichtung 104 wird dadurch vereinfacht. Dazu wird zunächst die erfasste räumliche Orientierung der Videobrille 105 der Kontrolleinheit 101 in Echtzeit übermittelt. Die Übermittlung kann mit der Kommunikationseinheit 102 erfolgen oder einer weiteren in Abbildung 1 nicht dargestellten Kommunikationseinheit erfolgen. In einer bevorzugten Ausführungsform erfolgt die Kommunikation mit Funk, womit sich der Vorteil ergibt, dass zu der Erfassungseinrichtung 104 kein Kabel im Operationssaal verlegt werden muss. Die räumliche Orientierung der Bildaufnahmeebene 110 kann über die Lageinformation des Roboterarms 111 und der relativen Orientierung des Stativs 106 zum Roboterarm 111 ermittelt werden. Die relative Orientierung des Stativs 106 kann dabei beispielsweise mit einem Drehwinkelgeber in Gelenk 1 12 ermittelt werden. In Analoger weise zu zuvor, kann aus den Orientierungsinformationen von Roboterarm 111 und Stativ 106 mittels linearer Transformationsmatrizen die räumliche Orientierung der Bildaufnahmeebene 110 ermittelt werden. Diese Funktionalität der Erfassungseinrichtung 104 kann wie zuvor Teil von Kontrolleinheit 101 sein, der in diesem Fall dazu eingerichtet ist aus der Position des Roboterarms 111 und des Stativs 106 die räumliche Orientierung der Bildaufnahmeebene 110 zu ermitteln. In einer alternativen Ausführungsform kann das optische System der Erfassungseinrichtung 104 durch am Stativ 106 angebrachten Markierungen die Orientierung des Stativs 106 und daraus in analoger weise wie zuvor für die Videobrille 105 beschrieben mittels Transformation die Orientierung der Bildaufnahmeebene 110 ermitteln.

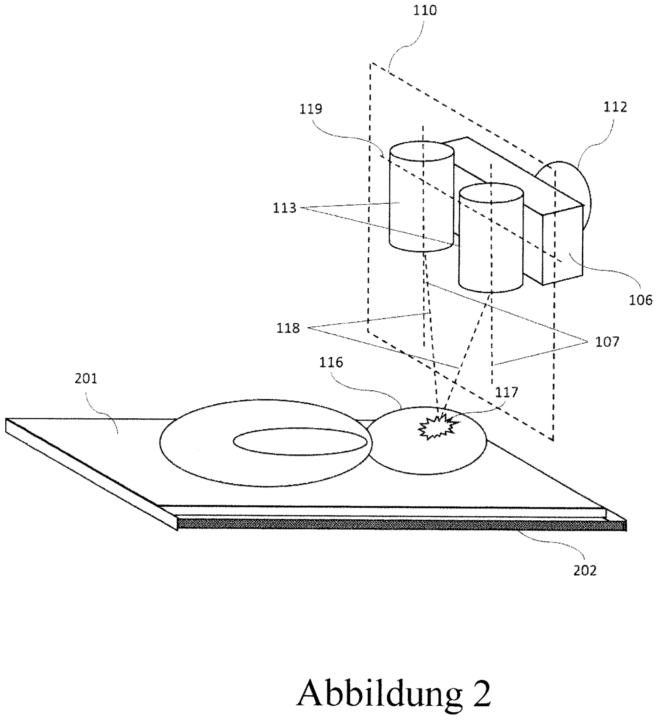

Abbildung 2 zeigt eine bevorzugte Ausführungsform des Stativs 106 für das erfindungsgemäße Stereomikroskop 100. Durch die zueinander parallel versetzten Bilderfassungseinheiten 113 sind die zugehörigen optischen Achsen 107 ebenfalls parallel zueinander. Damit wird es möglich den darunterliegenden abzubildenden Eingriffsbereich 117 am Patienten 116 aus zwei unterschiedlichen Richtungen 118 mit den Bilderfassungseinheiten 113 beobachten zu können. Diese dem menschlichen binokularen Sehen nachempfundene Bilderfassung ermöglicht es einen stereoskopischen Bildeindruck zu erzeugen, womit zusätzlich eine Tiefeninformation dem Benutzer 103 zur Verfügung gestellt werden kann. Diese zusätzliche Information ist von wesentlicher Bedeutung in der Mikrochirurgie und ermöglicht wesentlich sicherere mikrochirurgische Eingriffe als es ansonsten mit einem binokularen Mikroskop möglich wäre. Die beiden optischen Achsen 107 sind in Abbildung 1 parallel dargestellt, das ist für das erfindungsgemäße Stereomikroskop 100 jedoch nicht erforderlich. Jede Lage der Bilderfassungseinheiten 113 und damit der optischen Achsen 107 zueinander ist möglich, solange die optischen Achsen 107 eine Bildaufnahmeebene 110 bilden. In einer bevorzugten Ausführungsform sind die optischen Achsen 107 um einen Winkel in der Bildaufnahmeebene 110 zueinander geneigt. Damit lässt sich vorteilhaft eine größere Schärfentiefe als bei parallelen optischen Achsen 107 erzielen.

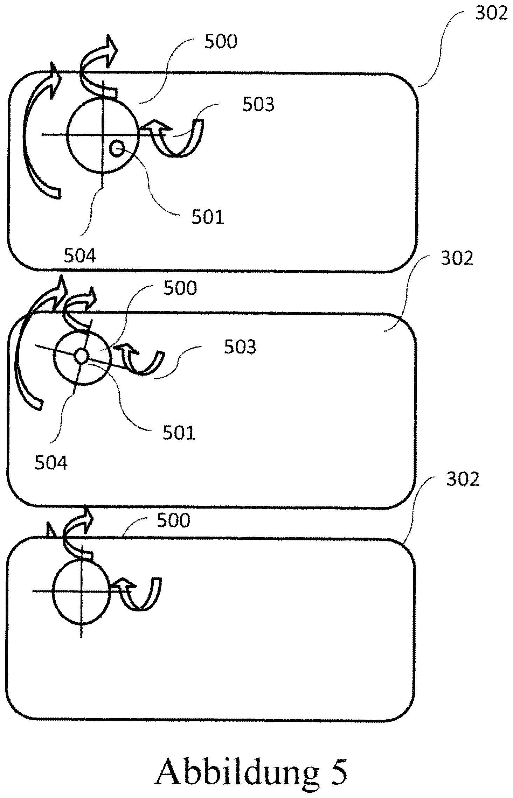

In einer weiteren vorteilhaften Ausführungsform können die optischen Achsen 107 an den Augenabstand der Benutzers 103 angepasst werden. Dazu ist das Stativ 106 mit Führungsschienen entlang der Längsachse 119 des Stativs 106 versehen. Die beiden Bilderfassungseinheiten 113 sind relativ zueinander entlang der Führungsschiene verschiebbar. In einer vorteilhaften Ausführungsform des erfindungsgemäßen Stereomikroskops 100 sind die Bilderfassungseinheiten 113 am Stativ 106 entlang der Führungsschiene derart verschiebbar, sodass sich jede der beiden Bilderfassungseinheiten 1 13 zu jedem Zeitpunkt im selben Abstand zum Mittelpunkt entlang der Längsachse der Führungsschiene befindet. Das hat den Vorteil, dass das stereoskopisch erfasste Bild des Eingriffsbereichs 117 bei verschieben der Bilderfassungseinheiten 113 nicht wandert, nur der Betrachtungswinkel und damit die Tiefeninformation verändert sich dem Benutzer 103 gegenüber. In einer weiteren vorteilhaften Ausführungsform sind die beiden Bilderfassungseinheiten 113 mit entlang der Führungsschiene beweglichen Zahnstangen verbunden, wobei die Zahnstangen derart angeordnet sind, dass die Zähne in einem im Mittelunkt der Führungsschiene befindlichen Zahnrad eingreifen und bei Drehung des Zahnrades sich gegenläufig zueinander bewegen. Dadurch wird vorteilhaft erreicht, dass beide Bilderfassungseinheiten 113 mit nur einem mit dem Zahnrad verbundenen Motor gleichzeitig relativ zueinander bewegt werden können und somit der Abstand der Bilderfassungseinheiten 113 verändert werden kann. In einer weiteren vorteilhaften Ausführungsform können die Bilderfassungseinheiten 113 in der Bildaufnahmeebene 110 drehbar sein. Die beiden Bewegungen der Bilderfassungseinheiten 113, d.h. der Bewegung entlang der Führungsschiene und der Drehbewegung in der Bildaufnahmeebene 110, können auch kombiniert werden. Dazu sind in vorteilhafter Weise die Bilderfassungseinheiten 113 derart zueinander beweglich mit dem Stativ 106 verbunden, um die stereoskopische Abbildung des Eingriffsbereichs 1 17 abhängig von der Vergrößerung und/oder dem Abstand der Bilderfassungseinheiten 113 vom Eingriffsbereich 117 und/oder dem Augenabstand des Benutzers 103 einzustellen. Dadurch kann für jede Betriebsstellung, d.h. Vergrößerung, Abstand der Bilderfassungseinheiten 113 vom Eingriffsbereich 117, Augenabstand der Benutzers 103, Beleuchtung und Vergrößerung/Verkleinerung die optimale Schärfentiefe und damit Tiefeninformation bei der stereoskopischen Bilderfassung gewährleistet werden. Die beweglichen Bilderfassungseinheiten 113 können dabei manuell bewegt werden mit einem von der Kontrolleinheit 101 dem Benutzer 103 geeignet bereitgestellten Kontrollsignal, etwa durch eine in der Videobrille 105 eingeblendete Information. Diese Ausführungsform wird anhand von Abbildung 5 weiter unten genauer beschrieben. Alternativ stellt die Kontrolleinheit 101 ein Kontrollsignal zur Ansteuerung einer Motoreinheit bereit, sodass die Bilderfassungseinheiten 113 automatisch bewegt und ausgerichtet werden können.

Das Stativ 106 ist über ein Gelenk 112 schwenkbar mit einem Roboterarm 1 1 1 verbindbar. Dadurch kann das Stativ 106 mit seinen empfindlichen Bilderfassungseinheiten 113 für den Transport vom schweren und unhandlichen Roboterarm 111 getrennt werden. Der Roboterarm 111 kann für den Transport an einer Haltevorrichtung angebracht sein. In einer weiteren bevorzugten Ausführungsform kann die Haltevorrichtung einen Wagen umfassen, der vor der Operation an dem Operationstisch 201 und möglichst nahe an den Eingriffsbereich 117 am Patienten 116 herangeführt werden kann. Der so in Position gebrachte Wagen kann mit herausklappbaren oder herausziehbaren Standfüßen zusätzlich abgestützt werden. In einer alternativen Ausführungsform kann der Wagen auch abgesenkt werden sodass die Wagenbodenfläche flächig stabil auf dem Boden des Operationssaales zum Aufliegen kommt. Eine geeignete Vorrichtung zum Absenken und Anheben ist beispielsweise eine manuell, hydraulisch, pneumatisch oder elektrisch angetriebene Scherenhebevorrichtung. Weitere äquivalente und bekannte Ausführungsformen sind ebenfalls möglich. In einer weiteren Ausführungsform kann die Haltervorrichtung C-förmig ausgebildete Klemmen zum Einhängen und Fixieren an den seitlich entlang des Operationstisches 201 verlaufenden Befestigungsleisten 202 aufweisen. Durch die Positionierung nahe am Eingriffsbereich 117 können kurze Armlängen des Roboterarms 111 realisiert werden, mit dem besonderen Vorteil, der erhöhten Schwingfestigkeit. Der Roboterarm 111 kann ein herkömmlicher Industrieroboterarm sein der derart modifiziert ist, sodass die hohen Anforderungen an die Positionierungsgenauigkeit und Schwingfestigkeit erfüllt werden können. Insbesondere die Schwingfestigkeit stellt ein wesentliches Kriterium dar, denn bereits kleinste Schwingbewegungen erscheinen in dem vergrößerten Bild des Eingriffsbereiches 117 ebenfalls vergrößert und führen zu unbrauchbar verwischten Bildern für den Benutzer 103. Zur Erlangung einer besonders hohen Stabilität und Schwingfestigkeit ist in einer bevorzugten Ausführungsform das Gewichtsverhältnis von Haltevorrichtung zu Roboterarm 111 samt Gelenk 112, Stativ 106 und Bilderfassungseinheiten 1 13 größer als 2 zu 1.

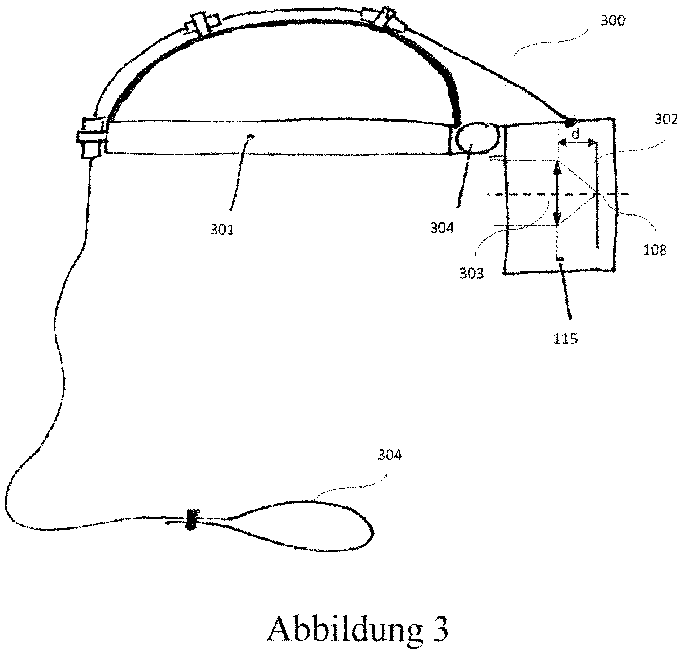

Abbildung 3 zeigt eine Videobrille 300 mit Stützvorrichtung 301 zum Halten der Videobrille 300 am Kopf des Benutzers 103. Die Bildwiedergabeeinheiten 115 sind über Gelenk 304 mit der Stützvorrichtung 301 verbunden. Die Videobrille 300 umfasst zusätzlich einen Seilzug 304 mit dem die Bildwiedergabeeinheit 115 mit dem Gelenk nach oben derart verschwenkbar ist um aus dem Blickfeld des die Videobrille tragenden Benutzers 103 herausgeführt zu werden. Im Folgenden wird die Videobrille 300 nur insoweit beschrieben, wie sie zum Verständnis der vorliegenden Erfindung erforderlich ist und jede aus dem Stand-der-Technik bekannte Videobrille 300 mit den folgend beschriebenen Merkmalen oder dazu äquivalenten Merkmalen kann für das erfindungsgemäße Stereomikroskop 100 verwendet werden, beispielsweise die Videobrille 300 welche Gegenstand einer weiteren anhängigen nationalen österreichischen Patentanmeldung der Erfinder mit der Veröffentlichungsnummer AT 519845. Jede Bildwiedergabeeinheit 115 umfasst ein Display 302 und eine Sammellinse 303. Die beiden Bildanzeigevorrichtungen 302 der beiden Bildwiedergabeeinheiten sind in einer Ebene, die Bildebene 109 bildend, angeordnet und können entweder als ein einziges Display, wobei das auf dem Display ausgegebene Bild für je eine Bildwiedergabeeinheit 115 geteilt wird, oder als zwei getrennte Displays ausgebildet sein. Die Sammellinse 303 ist von der Bildebene 109 in Richtung der optischen Achse 108 in etwa mit der Brennweite d der Sammellinse 303 beabstandet. Damit wird in bekannter weise eine Abbildung im menschlichen Auge möglich, wenn das Auge sich entspannt und auf unendlich fokussiert ist. In einer Ausführungsform ist die Bildwiedergabeeinheit 115 dazu eingerichtet an den Augenabstand des Benutzers 103 anpassbar zu sein, indem die optische Achsen 108 mit den optischen Achsen der Augen des Benutzers 103, wenn der Benutzer 103 geradeaussieht und die Augen auf unendlich fokussiert, in Übereinstimmung zu bringen. In einer Ausführungsform wird das durch zueinander bewegliche Bildwiedergabeeinheiten 115 erreicht. In einer weiteren vorteilhaften Ausführungsform wird das ohne weitere mechanische Elemente in der Videobrille 300 dadurch realisiert, dass die Bildausgabe der Bildwiedergabeeinheiten 115 in der Bildebene 109 zueinander verschoben wird. Das erfolgt z.B. dadurch, dass die Bildausgabe durch das Display und das auszugebende Bild um einen oder mehrere Bildpunkte verschoben ausgegeben wird.

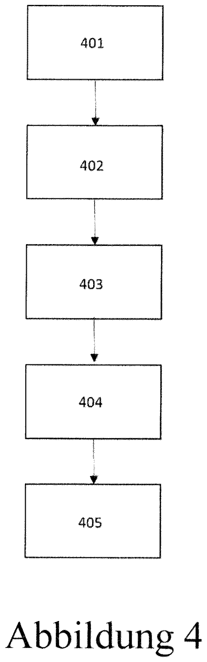

Abbildung 4 zeigt ein Flussdiagramm zur Steuerung des erfindungsgemäßen Stereomikroskops 100. Zur Inbetriebnahme des Stereomikroskops 100 wird zunächst das Stereomikroskop 100 an den Operationstisch 201 mit dem Wagen herangeführt und mit den zuvor Beschriebenen Einrichtungen stabil am Operationssaalboden abgestellt. Anschließend wird das Stativ 106 mit den beiden Bilderfassungseinheiten 113 über den zu beobachtenden Eingriffsbereich 117 mit dem Roboterarm 111 positioniert. Dazu wird der Roboterarm 111 mit der Kontrolleinheit 101 angesteuert. Die Kommunikationseinheit 102 kommuniziert die von den beiden Bilderfassungseinheiten 1 13 erfassten Bilder des Eingriffsbereichs 1 17 in Echtzeit an die Bildwiedergabeeinheiten 115 zur stereoskopischen Anzeige. Dabei wird das erfasste Bild von je einer der beiden Bilderfassungseinheiten 113 an je eine der beiden Bildwiedergabeeinheiten 115 zur Anzeige übermittelt. Damit wird erreicht, dass das stereoskopisch erfasste Bild des Eingriffsbereiches 1 17 je einem Auge des die Videobrille 105 tragenden Benutzers 103 angezeigt wird und dem Benutzer 103 ein stereoskopischer Bildeindruck des Eingriffsbereichs 117 angezeigt werden kann.

Zur erfindungsgemäßen Steuerung des Stereomikroskops 100 ist Kontrolleinheit 101 eingerichtet um die Schritte, die Anhand der Abbildung 4 im Folgenden näher erläutert werden, auszuführen. In Schritt 401 erfolgt die Erfassung der Orientierung der Bildwiedergabeebene 114 und der Bildebene 119. Das kann mit den zuvor beschriebenen Verfahren und Vorrichtungen erfolgen, beispielsweise mit Hilfe der Erfassungseinrichtung 104 und Bereitstellung an die Kontrolleinheit 101 zur weiteren Verarbeitung. Die Orientierung der Bildwiedergabeebene 114 kann dann beispielsweise durch eine Ebenengleichung der Form *E*1: *a*₁ · *x* = *d*₁ mit den dreidimensionalen Vektoren *a*₁ und *x,* sowie dem Skalar *d*₁ und wobei "·" das vektorielle Skalarprodukt bezeichnet dargestellt werden. Die so erfasste Orientierung durch Erfassungseinrichtung 104 kann beispielsweise mit Kommunikationseinheit 102 an die Kontrolleinheit 101 übermittelt werden. In gleicher Weise kann Bildebene 109 durch die Ebenengleichung E: *a* · *x* = d dargestellt werden. In Schritt 402 erfolgt die Erfassung der Orientierung der Bildaufnahmeebene 119. Wie zuvor kann die Ebenengleichung durch E2: *a*₂ · *x* = *d₂* dargestellt werden. In Schritt 403 ermittelt die Kontrolleinheit 101 eine erste Schnittline von der Bildebene 109 in der Bildwiedergabeebene 114. Dazu berechnet Kontrolleinheit 101 beispielsweise das Vektorprodukt von *O*1: *a*₁ × *a*. Es ist nicht notwendig die vektorielle Schnittgeradengleichung vollständig zu berechnen, der durch das Vektorprodukt errechnete Richtungsvektor 01 ist ausreichend. In Schritt 404 ermittelt die Kontrolleinheit 101 eine zweite Schnittlinie von der Bildaufnahmeebene 110 in der Bildwiedergabeebene 114. Das kann in der Kontrolleinheit 101 beispielsweise in derselben Weise wie zuvor berechnen indem das Vektorprodukt von *O*2: *a*₂ × *a* gebildet wird. Abermals genügt es den Richtungsvektor 02 zu berechnen, eine vollständige Berechnung der Geradengleichung ist nicht notwendig. Schließlich kann in Schritt 405 von Kontrolleinheit 101 das Kontrollsignal zum Verschwenken des Stativs 106 ermittelt und ausgegeben werden, sodass die erste und zweite Schnittlinie parallel gebracht werden können. Das Kontrollsignal könnte beispielsweise eine Winkelinformation *α* und eine Drehebene *D* sein. Den Drehwinkel *α* könnte Kontrolleinheit 101 beispielsweise durch das Bilden des Skalarproduktes aus den zuvor ermittelten Richtungsvektoren 01 und 02 berechnen: *α* = arccos(*O*₁ · *O*₂/(|*O*₁| · |*O*₂|)). Die Drehebene D kann Kontrolleinheit 101 beispielsweise aus dem Vektorprodukt der zuvor ermittelten Richtungsvektoren 01 und 02 berechnen: *D:* (*O*₁ × *O*₂) · *x* = (*O*₁ × *O*₂) · *O̅P̅,* wobei *O̅P̅* die Koordinaten des Drehpunktes im Gelenk 1 12 bezeichnet um den das Stativ 106 verschwenkt werden kann. Das so errechnete Kontrollsignal kann wie zuvor beschrieben von Kontrolleinheit 101 derart durch Einblenden in zumindest einer Bildwiedergabeeinheit 115 erfolgen, um dem Benutzer 103 die manuelle Verschwenkung des Stativs 106 anzuzeigen. In einer weiteren bevorzugten Ausführungsform kann das Kontrollsignal derart bereitgestellt werden, um eine Motoreinheit anzusteuern die das Stativ 106 in der berechneten Drehebene D um den Drehwinkel a zu verschwenken. Zur Ausführung der Schritte 401 bis 405 kann Kontrolleinheit 101 als Computer ausgeführt sein.

Abbildung 5 zeigt schematisch das Display 302 einer Bildwiedergabeeinheit 115 mit der Überblendung durch ein beispielhaftes Kontrollsignal um dem Benutzer die manuelle Verschwenkung des Stativs anzuzeigen. Die optische Anzeige des Kontrollsignals 500 überblendet beispielsweise in der oberen linken Ecke den Bildbereich. Es umfasst eine in der Abbildung 5 horizontal dargestellte Achse 503 und eine vertikale Achse 504 die rechtwinkelig aufeinander stehen und ein Kreuz bilden. Punkt 501 gibt die aktuelle relative Orientierung des Stativs 106 an. Ist Punkt 501 unter der horizontalen Achse 503, so wird dem Benutzer damit angezeigt, dass eine Rotation um die Längsachse (X-Achse) des Stativs 106 wie mit dem Doppelpfeil an der horizontalen Achse 503 durchzuführen ist und zwar solange, bis Punkt 501 auf der horizontalen Achse 503 zum Liegen kommt, dann ist Stativ 106 entlang der Längsachse, angezeigt durch Achse 503 ausgerichtet. Befindet sich Punkt 503 oberhalb von Achse 503 ist die Rotation um die Längsachse (X-Achse) in entgegengesetzter Richtung von Benutzer 103 auszuführen. Analog dazu lässt sich Stativ 106 entlang Achse 504 ausrichten, wobei hier eine Rotation um die Querachse (Y-Achse) des Stativs 106 auszuführen ist. Der dritte Rotationswinkel um die Hochachse (Z-Achse) des Stativs 106 kann über eine Verdrehung des gesamten Kreuzes, gebildet aus den beiden Achsen 503 und 504 dem Benutzer 103 angezeigt werden, wie beispielsweise in der Mittleren schematischen Darstellung in Abbildung 5 gezeigt. Erst wenn Achse 503 dem Benutzer 103 horizontal erscheint, ist Stativ 106 auch entsprechend der Hochachse des Stativs 106 ausgerichtet. Diese vollständige Ausrichtung des Stativs 106 ist in der unteren Abbildung dargestellt.

## Patentansprüche

1. Stereomikroskop (100) zur Verwendung bei mikrochirurgischen Eingriffen umfassend einen Roboterarm (111), ein Stativ (106) schwenkbar über ein Gelenk (112) mit dem Roboterarm (111 ) verbindbar,
zwei mit dem Stativ (106) derart verbindbare optische Bilderfassungseinheiten (113), um ein stereoskopisches Bild des während der Benutzung abzubildenden Eingriffsbereichs (117) am Patienten erfassen zu können und durch die zwei optischen Achsen (107) der Bilderfassungseinheiten (113) eine Bildaufnahmeebene (110) definierend,
eine Videobrille (105) umfassend zwei optische Bildwiedergabeeinheiten (115) mit je einer optischen Achse (108) und einer Anzeige zur Wiedergabe eines Bildes, welche gemeinsam eine Bildebene (109) definieren,
wobei die optischen Bildwiedergabeeinheiten (115) derart angeordnet sind zur Erzeugung eines stereoskopischen Bildeindruckes einem die Videobrille (105) tragenden Benutzers (103) und durch die zwei optischen Achsen (108) der optischen Bildwiedergabeeinheiten (115) eine Bildwiedergabeebene (114) definierend,
eine Erfassungseinrichtung (104) zur Ermittlung der räumlichen Orientierung der Videobrille (105), Bildwiedergabeebene (114), Bildebene (109) und der Bildaufnahmeebene (110) und
eine Kontrolleinheit (101) eingerichtet, ein Kontrollsignal zum Verschwenken des Stativs (106) durch
- Ermitteln einer ersten Schnittlinie von der Bildebene (109) in der Bildwiedergabeebene (114)
- Ermitteln einer zweiten Schnittlinie von der Bildaufnahmeebene (110) in der Bildwiedergabeebene (114)
- Ermitteln und Ausgabe des ermittelten Kontrollsignals zum Verschwenken des Stativs (106)
bereitzustellen, sodass die Schnittlinien in der Bildwiedergabeebene (114) von der Bildebene (109) und der Bildaufnahmeebene (110) parallel gebracht werden können.

2. Stereomikroskop (100) nach Anspruch 1, wobei das Kontrollsignal optisch in zumindest einer Bildwiedergabeeinheit (115) eingeblendet wird und geeignet ist, dem Benutzer (103) die manuelle Verschwenkung des Stativs (106) um Gelenk (112) anzuzeigen.

3. Stereomikroskop ( 100) nach Anspruch 1, wobei das Stativ (106) mit einer Motoreinheit um Gelenk (112) schwenkbar ist und das Kontrollsignal ausgebildet ist, die Motoreinheit anzusteuern.

4. Stereomikroskop (100) nach einem der vorhergehenden Ansprüche, wobei die zwei Bildwiedergabeeinheiten (115) ein gemeinsames Display umfassen, dessen Anzeigebereich derart in zwei Hälften geteilt ist, um je einem Auge des die Videobrille (105) tragenden Benutzers (103) ein Bild anzeigen zu können.

5. Stereomikroskop (100) nach einem der vorhergehenden Ansprüche, wobei die Erfassungseinrichtung (104) zur Ermittlung der räumlichen Orientierung der Videobrille (105) und/oder Bildaufnahmeebene (110) ein optisches Objekterfassungssystem umfasst.

6. Stereomikroskop (100) nach einem der Ansprüche 1 bis 4, wobei die Erfassungseinrichtung (104) zur Ermittlung der räumlichen Orientierung der Videobrille (105) einen mit der Videobrille (105) verbindbaren Orientierungssensor umfasst.

7. Stereomikroskop ( 100) nach Anspruch 6, wobei der Orientierungssensor ein Lagesensor, ein Positionssensor, ein Beschleunigungssensor oder ein Inertialmesssystem ist.

8. Stereomikroskop (100) nach einem der vorhergehenden Ansprüche, zusätzlich aufweisend eine Kommunikationseinheit (102) eingerichtet, um in Echtzeit die stereoskopisch erfassten Bilder von den Bilderfassungseinheiten (113) an die Bildwiedergabeeinheiten (115) zur stereoskopischen Anzeige zu übermitteln.

9. Stereomikroskop (100) nach Anspruch 8, wobei der Zeitversatz in der Echtzeitkommunikation kleiner als 50 Millisekunden beträgt.

10. Stereomikroskop (100) nach einem der Ansprüche 8 oder 9, wobei die Kommunikationseinheit (102) eingerichtet ist, die Bilder per drahtloser Kommunikation übermitteln zu können.

11. Stereomikroskop (100) nach einem der vorhergehenden Ansprüche, wobei das Stativ (106) eine Schnellwechselplatte umfasst, mit dem die Bildwiedergabeeinheiten (115) verbindbar sind.

12. Stereomikroskop (100) nach einem der vorhergehenden Ansprüche, wobei die Bilderfassungseinheiten (113) derart zueinander beweglich sind, um die stereoskopische Abbildung des Eingriffsbereichs (117) abhängig von der Vergrößerung und/oder dem Abstand der Bilderfassungseinheiten (113) vom Eingriffsbereich (117) und/oder dem Augenabstand des Benutzers (103) einzustellen.

13. Stereomikroskop (100) nach Anspruch 12, wobei die Bilderfassungseinheiten (113) in der Bildaufnahmeebene (110) drehbar sind, sodass die optischen Achsen (107) zueinander einen Winkel kleiner 180° einschließen.

14. Stereomikroskop (100) nach einem der Ansprüche 12 oder 13, wobei der Abstand der beiden Bilderfassungseinheiten (113) zueinander abhängig vom Abstand der Bilderfassungseinheiten (113) vom Eingriffsbereich (117) eingestellt wird.

15. Verfahren zum Steuern eines Stereomikroskops (100) nach einem der vorhergehenden Ansprüche, wobei die Kontrolleinheit (101) eingerichtet ist, die folgenden Schritte auszuführen:
- Erfassung der Orientierung der Videobrille (105), Bildwiedergabeebene (114) und Bildebene (109)
- Erfassung der Orientierung der Bildaufnahmeebene (110)
- Ermitteln einer ersten Schnittlinie von der Bildebene (109) in der Bildwiedergabeebene (114)
- Ermitteln einer zweiten Schnittlinie von der Bildaufnahmeebene (110) in der Bildwiedergabeebene (114)
- ermittelten und Ausgabe des ermittelten Kontrollsignals zum Verschwenken des Stativs (106), sodass die erste und zweite Schnittlinie parallel gebracht werden können.

## Claims

1. Stereo microscope (100) for use in microsurgical surgeries, comprising a robot arm (111), a stand (106) pivotally connectable through a joint (112) to the robot arm (111),
two optical image acquisition units (113) configured to connect to the stand (106) in such a way that a stereoscopic image of a surgery area (117) can be captured during use on a patient and an image acquisition plane (110) is defined through the two optical axis (107) of the optical image acquisition units (113),
video glasses (105) comprising two optical image reproduction units (115) each having an optical axis (108) and a display for reproducing an image, which together define an image plane (109),
wherein the optical image reproduction units (115) are arranged to produce a stereoscopic image impression for a user (103) wearing the video glasses (105), and the two optical axis (108) of the optical image reproduction units (115) define an image reproduction plane (114),
a detection device (104) configured to determine the spatial orientation of the video glasses (105), the image reproduction plane (114), the image plane (109) and the image acquisition plane (110), and
a control unit (101) adapted to provide a control signal for pivoting the stand (106) by
- determining a first intersection line of the image plane (109) in the image reproduction plane (114);
- determining a second intersection line of the image acquisition plane (110) in the image reproduction plane (114); and
- determining and output of the determined control signal for pivoting the stand (106)
such that the intersection lines in the image reproduction plane (114) of the image plane (109) and the image acquisition plane (110) are made parallel.

2. Stereo microscope (100) according claim 1, wherein the control signal is optically displayed in at least one image reproduction unit (115) and adapted for displaying to the user the manual pivoting of the stand (106) around the joint (112).

3. Stereo microscope (100) according claim 1, wherein the stand (106) is pivotable with a motor unit around the joint (112) and the control signal is adapted to control the motor unit.

4. Stereo microscope (100) according to any one of the preceding claims, wherein the two image reproduction units (115) comprise a common display, the display area of which is divided into two halves in order to be able to display one image each to one eye of the user (103) wearing the video glasses (105).

5. Stereo microscope (100) according to any one of the preceding claims, wherein the detection device (104) configured to determine the spatial orientation of the video glasses (105) and/or image acquisition plane (110) comprises an optical object detection system.

6. Stereo microscope (100) according to any one of the claims 1 to 4, wherein the detection device (104) configured to determine the spatial orientation of the video glasses (105) comprises an orientation sensor connectable to the video glasses (105).

7. Stereo microscope (100) according to claim 6, wherein the orientation sensor is an orientation sensor, a position sensor, an acceleration sensor or an inertial measurement system.

8. Stereo microscope (100) according to any one of the preceding claims, further comprising a communication unit (102), adapted for real time transfer of the stereoscopic acquired images of the image acquisition units (113) to the image reproduction units (115) for stereoscopic displaying.

9. Stereo microscope (100) according to claim 8, wherein the time delay in the reals time communication is less than 50 milliseconds.

10. Stereo microscope (100) according to claim 8 or 9, wherein the communication unit (102) is adapted to transfer the images through wireless communication.

11. Stereo microscope (100) according to any one of the preceding claims, wherein the stand (106) comprises a quick-release plate with which the image acquisition units (115) are connectable.

12. Stereo microscope (100) according to an one of the preceding claims, wherein the image acquisition units (113) are movable relative to one another in order to set the stereoscopic image of the surgery area (117) depending on the magnification and/or the distance of the image acquisition units (113) from the surgery area (117) and/or the pupillary distance of the user (103).

13. Stereo microscope (100) according to claim 12, wherein the image acquisition units (113) are rotatable in the image acquisition plane (110), so that the optical axes (107) form an angle of less than 180° to one another.

14. Stereo microscope (100) according to claim 12 or 13, wherein the distance between the two image acquisition units (113) from one another is dependent on the distance of the image acquisition units (113) from the surgery area (117).

15. Method for controlling a stereo microscope (100) according to any one of the preceding claims, wherein the control unit (101) is adapted to carry out the following steps:
- detecting the orientation of the video glasses (105), the image reproduction plane (114) and the image plane (109);
- detecting the orientation of the image acquisition plane (110);
- determining a first intersection line of the image plane (109) in the image reproduction plane (114);
- determining a second intersection line of the image acquisition plane (110) in the image reproduction plane (114);
- determining and output of the determined control signal for pivoting the stand (106) so that the first and second intersection lines can be brought parallel.

## Revendications

1. Stéréomicroscope (100) pour une utilisation en microchirurgie comprenant un bras de robot (111), un trépied (106) pouvant être relié de manière pivotante au bras de robot (111) via une articulation (112),
deux unités d'acquisition d'images optiques (113) pouvant être connectées au trépied (106) de manière à pouvoir acquérir une image stéréoscopique de la zone d'intervention (117) du patient à imager pendant l'utilisation et définissant un plan d'acquisition d'images (110) à travers les deux axes optiques (107) des unités d'acquisition d'images (113),
des lunettes vidéo (105) comprenant deux unités de reproduction d'image optique (115) ayant chacune un axe optique (108) et un affichage pour reproduire une image, qui définissent ensemble un plan d'image (109),
dans lequel les unités de reproduction d'image optique (115) sont disposées de manière à créer une impression d'image stéréoscopique pour un utilisateur (103) portant les lunettes vidéo (105) et définissant un plan de reproduction d'image (114) à travers les deux axes optiques (108) des unités de reproduction d'image optique (115),
un dispositif de détection (104) pour déterminer l'orientation spatiale des lunettes vidéo (105), du plan de reproduction d'image (114), du plan d'image (109) et du plan de capture d'image (110) et
une unité de contrôle (101), un signal de contrôle pour le pivotement du trépied (106) par
- Détermination d'une première ligne de coupe à partir du plan d'image (109) dans le plan de reproduction d'image (114)
- Détermination d'une deuxième ligne de coupe à partir du plan de capture d'image (110) dans le plan de reproduction d'image (114)
- Détermination et sortie du signal de contrôle déterminé pour le pivotement du trépied (106)
afin que les lignes de coupe dans le plan de reproduction d'image (114) puissent être amenées en parallèle à partir du plan d'image (109) et du plan de capture d'image (110).

2. Stéréomicroscope (100) selon la revendication 1, dans lequel le signal de contrôle est affiché optiquement dans au moins une unité de reproduction d'image (115) et est adapté pour indiquer à l'utilisateur (103) le pivotement manuel du trépied (106) autour de l'articulation (112).

3. Stéréomicroscope (100) selon la revendication 1, dans lequel le trépied (106) peut pivoter avec une unité de moteur autour de l'articulation (112) et le signal de contrôle est conçu pour commander l'unité de moteur.

4. Stéréomicroscope (100) selon l'une quelconque des revendications précédentes, dans lequel les deux unités de reproduction d'image (115) comprennent un affichage commun dont la zone d'affichage est divisée en deux moitiés de manière à pouvoir afficher une image à un œil de l'utilisateur (103) portant les lunettes vidéo (105).

5. Stéréomicroscope (100) selon l'une quelconque des revendications précédentes, dans lequel le dispositif de détection (104) comprend un système de détection d'objet optique pour déterminer l'orientation spatiale des lunettes vidéo (105) et/ou du plan de capture d'image (110).

6. Stéréomicroscope (100) selon l'une quelconque des revendications 1 à 4, dans lequel le dispositif de détection (104) pour déterminer l'orientation spatiale des lunettes vidéo (105) comprend un capteur d'orientation pouvant être connecté aux lunettes vidéo (105).

7. Stéréomicroscope (100) selon la revendication 6, dans lequel le capteur d'orientation est un capteur de position, un capteur de position, un capteur d'accélération ou un système de mesure inertielle.

8. Stéréomicroscope (100) selon l'une quelconque des revendications précédentes, comprenant en outre une unité de communication (102) configurée pour transmettre en temps réel les images capturées stéréoscopiquement des unités d'acquisition d'images (113) aux unités de reproduction d'images (115) pour un affichage stéréoscopique.

9. Stéréomicroscope (100) selon la revendication 8, dans lequel le décalage temporel est inférieur à 50 millisecondes dans la communication en temps réel.

10. Stéréomicroscope (100) selon l'une quelconque des revendications 8 ou 9, dans lequel l'unité de communication (102) est adaptée pour transmettre les images par communication sans fil.

11. Stéréomicroscope (100) selon l'une quelconque des revendications précédentes, dans lequel le trépied (106) comprend une plaque à changement rapide à laquelle les unités de reproduction d'image (115) peuvent être connectées.

12. Stéréomicroscope (100) selon l'une quelconque des revendications précédentes, dans lequel les unités d'acquisition d'images (113) sont mobiles les unes par rapport aux autres de manière à régler l'image stéréoscopique de la zone d'intervention (117) en fonction du grossissement et/ou de la distance des unités d'acquisition d'images (113) par rapport à la zone d'intervention (117) et/ou de la distance oculaire de l'utilisateur (103).

13. Stéréomicroscope (100) selon la revendication 12, dans lequel les unités d'acquisition d'images (113) peuvent être tournées dans le plan de capture d'image (110) de sorte que les axes optiques (107) forment entre eux un angle inférieur à 180°.

14. Stéréomicroscope (100) selon l'une quelconque des revendications 12 ou 13, dans lequel la distance entre les deux unités d'acquisition d'images (113) l'une par rapport à l'autre est réglée en fonction de la distance entre les unités d'acquisition d'images (113) et la zone d'intervention (117).

15. Procédé de commande d'un stéréomicroscope (100) selon l'une quelconque des revendications précédentes, dans lequel l'unité de commande (101) est agencée pour effectuer les étapes suivantes :
- Détection de l'orientation des lunettes vidéo (105), du plan de reproduction d'image (114) et du plan d'image (109)
- Détection de l'orientation du plan de capture d'image (110)
- Détermination d'une première ligne de coupe à partir du plan d'image (109) dans le plan de reproduction d'image (114)
- Détermination d'une deuxième ligne de coupe à partir du plan de capture d'image (110) dans le plan de reproduction d'image (114)
- détecté et sortie du signal de contrôle détecté pour le pivotement du trépied (106), de sorte que les première et deuxième lignes de coupe puissent être amenées en parallèle.
